# EUROPEAN PATENT SPECIFICATION

(11) **EP 2 559 389 B1**
(45) Date of publication and mention of the grant of the patent: **03.04.2013**
(21) Application number: 11178034.2
(22) Date of filing: 18.08.2011
(51) Int. Cl.: A61B 17/70

(54) **Polyaxial bone anchoring device**
Polyaxiale Knochenverankerungsvorrichtung
Dispositif d'ancrage d'os polyaxial

(43) Date of publication of application: 20.02.2013
(73) Proprietor: Biedermann Technologies GmbH & Co. KG, 78166 Donaueschingen (DE)
(72) Inventor: Biedermann, Timo, 78647 Trossingen (DE); Biedermann, Lutz, 78048 VS-Villingen (DE)
(74) Representative: Prüfer & Partner GbR European Patent Attorneys

(56) References cited:
- EP-A1- 2 221 012
- US-A1- 2003 167 058
- US-A1- 2004 138 662
- US-A1- 2004 204 711
- US-A1- 2010 292 740

## Description

The invention relates to a polyaxial bone anchoring device for anchoring a stabilization rod in a bone or in a vertrebra. The bone anchoring device includes an anchoring element, a receiving part for receiving a head of the bone anchoring element and for receiving a stabilization rod to be connected to the anchoring element. The anchoring element is pivotably connected to the receiving part and can be locked at an angle by exerting pressure onto the head via a pressure element that is arranged in the receiving part. The pressure element and the receiving part are configured to cooperate in such a way that the pressure element frictionally clamps the head to maintain a desired angular position before locking the polyaxial bone anchoring device.

US 5,716,356 describes a polyaxial bone screw including a screw element and a receiving part which is pivotably connected to the screw element and a pressure element to exert pressure onto the head of the screw element to lock the angle between the screw element and the receiving part. The receiving part has a U-shaped channel for receiving a stabilization rod. The pressure element comprises a cylindrical recess, which is to be aligned with the U-shaped channel to receive the rod therein. In order to hold the pressure element in a position aligned with the U-shaped channel, the position of the pressure element is fixed by crimping through bores provided in the receiving part.

When the head of the bone anchoring element is freely pivotable with respect to the receiving part before locking the head in a final angular position, the alignment of the receiving part and the insertion of the rod may be difficult in more complex clinical applications, for example, when a multitude of bone anchors have to be connected to the rod.

US 7,604,656 (Shluzas) describes a fastener engageable with a bone portion to connect a longitudinal member to the bone portion. The housing that receives the fastener also receives a spacer, which is engageable with the fastener and the longitudinal member. In one embodiment, the spacer is urged by a pin member into frictional engagement with the fastener and with the housing.

US 2003/0167058 A1 discloses an apparatus comprising a longitudinal member connectable with a bone portion, a fastener, a housing, a spacer, a member fixedly connected to said housing and a clamping mechanism.

US 2010/0292740 A1 discloses a bone stabilizer assembly comprising a fixation element, a coupling element and a bottom saddle movably mounted in the coupling element below a stabilizer rod.

EP 2 221 012 A1 discloses a receiving part for receiving a rod for coupling the rod to a bone anchoring element, the receiving part including a receiving part body with a channel for receiving the rod, and a accommodation space, a head and a pressure element.

US 2004/0138662 A1 discloses a bone fastener assembly comprising a collar, a ring coupled to the collar, wherein the ring comprises two or more seats, a bone fastener comprising a shank, a head, and two or more splines on the head of the bone fastener, wherein at least one of the splines is configured to couple to at least one of the seats to inhibit separation of the bone fastener from the collar, and wherein the ring is configured to allow polyaxial movement of the collar relative to the shank.

US 2004/0204711 discloses a swivel head bone screw comprising a head having structure associated therewith for receiving a rod member, said head having a body, a shank having a lower threaded portion and an upper portion operably received within said head; said shank being swivelable about said upper portion relative to said head, said head body having a threaded aperture and a set screw operably received in said aperture.

It is an object of the invention to provide a polyaxial bone anchoring device and a method for manufacturing the same which allows an improved handling during surgery and which can be manufactured in a simple manner.

The object is solved by a polyaxial bone anchoring device according to claim 1. Further developments are given in the dependent claims.

With the polyaxial bone anchoring device a temporary clamping of the head in a desired angular position with respect to a receiving part without locking the head can be achieved. This allows to maintain the receiving part in an adjustable angular position. In this condition, the pressure element exerts a preload onto the head in which the head is not locked but prevented from freely pivoting. When the head is temporarily clamped, the alignment of the receiving part with respect to the rod and the insertion of the rod is facilitated, in particular in a situation in which a multitude of bone anchors have to be connected to the rods.

When the rod is already inserted into the receiving part, adjustments of the rod are still possible without completely unlocking the head.

The polyaxial bone anchoring device comprises only few parts and which are of simple design and wherein relatively large manufacturing tolerances are possible. The mechanism to frictionally maintain the head before locking is free from any spring members or portions. This facilitates the manufacturing of the polyaxial bone anchoring device. Furthermore, existing receiving parts and pressure elements can be used without modifying their form. For example, it is possible to change existing crimp bores to threaded through bores.

The amount of preload exerted onto the head by the pressure member can be exactly predefined in a simple manner by selecting the position and shape of the bores and the shape of the set screw and furthermore by the force applied by the set screw that is dependent on its depth of screwing in.. By means of the set screws a stepless adjustability of the pressure which is exerted on the screw head is achievable. By screwing in the set screw tightly a polyaxial screw can be used as a monoaxial screw. The friction force exerted by the set screws is reversible by loosening the set screws.

The polyaxial bone anchoring device can be provided to the surgeon in a pre-assembled manner, in which the pressure element is actually and rotationally fixed by the set screw to such an extent that it can not fall out or be rotated out of its aligned position. This allows a save insertion by the surgeon. It is also possible to provide receiving parts with pre-mounted set screws which allows the surgeon to introduce the desired bone screw, to insert the pressure element into the receiving part and to lock the polyaxial bone anchoring device by means of the set screw or the set screws.

The receiving part and the pressure element can be manufactured in series at low costs.

Further features and advantageous of the invention will become apparent from the description of the embodiments by means of the accompanying drawings.

In the drawings:
- Fig. 1: shows a perspective exploded view of the polyaxial bone anchoring device according to a first embodiment.
- Fig. 2: shows the polyaxial bone anchoring device of Fig. 1 in an assembled state.
- Fig. 3: shows a cross-sectional view of the polyaxial bone anchoring device in an assembled state before final locking of the head with two non-assembled set screws.
- Fig. 4: shows a cross-sectional view of the polyaxial bone anchoring device in an assembled state after locking the head.
- Fig. 5: shows a perspective top view of a pressure element.
- Fig. 6: shows a perspective bottom view of the pressure element.
- Fig. 7: shows a cross-sectional view of the pressure element.
- Fig. 8: shows a top view of the pressure element.
- Fig. 9: shows a perspective view of a set screw.
- Fig. 10: shows a side view of the set screw.
- Fig. 11: shows a top view of the set screw
- Fig. 12: shows a perspective view of a polyaxial bone anchoring device according to a second embodiment in an assembled state.
- Fig. 13: shows a cross-sectional view of the polyaxial bone anchoring device of Fig. 5.
- Fig. 14: shows a cross-sectional view of a polyaxial bone anchoring device according to a third embodiment in an assembled state.
- Fig. 15: shows a cross-sectional view of a polyaxial bone anchoring device according to a fourth embodiment in an assembled state.
- Fig. 16: shows a perspective exploded view of a polyaxial bone anchoring device according to a fifth embodiment.
- Fig. 17: shows the polyaxial bone anchoring device of Fig. 16 in an assembled state.
- Fig. 18: shows a cross-sectional view of the polyaxial bone anchoring device according to the fifth embodiment in an assembled state.
- Fig. 19: shows a perspective view of a polyaxial bone anchoring device according to a sixth embodiment in an assembled state.
- Fig. 20: shows a cross-sectional view of the polyaxial bone anchoring device according to the sixth embodiment in an assembled state.

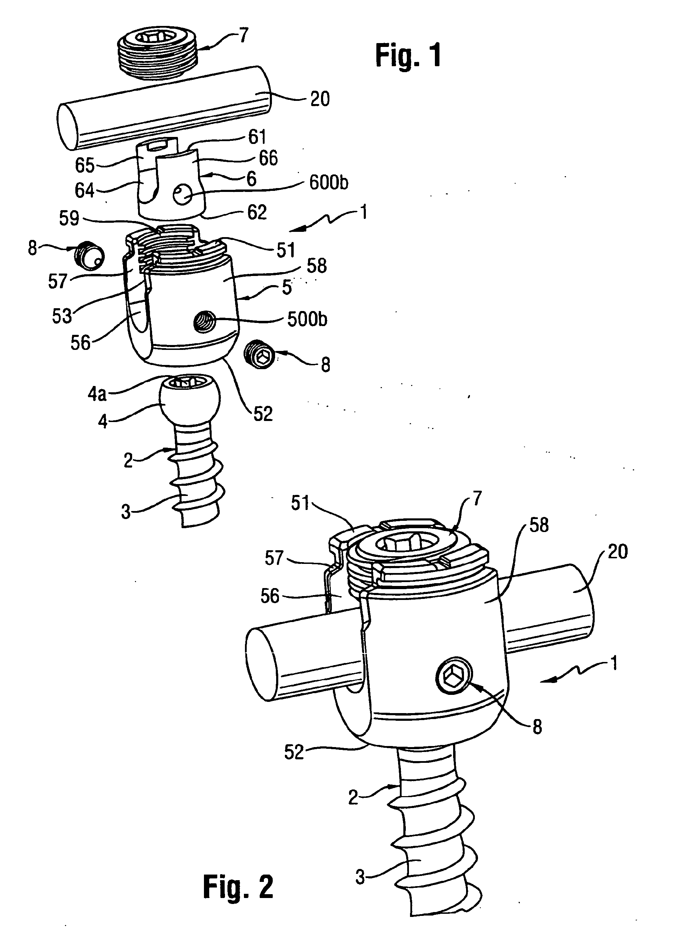

A polyaxial bone anchoring device 1 according to a first embodiment as shown in Figs. 1 to 4 includes a bone anchoring element 2 in the form of a screw member 2 having a threaded shaft 3 and a head 4. The head 4 is generally spherical and includes a recess 4a at its free end for engagement with a tool to insert the threaded shaft 3 into the bone. The bone anchoring device 1 further includes a receiving part 5 for connecting the screw member 2 to a rod 20. A pressure element 6 is arranged in the receiving part 5 on top of the head 4. For securing the rod 20 in the receiving part 5 and for exerting pressure onto the head 4, a locking device , for example an inner screw 7, which cooperates with the receiving part 5, is provided.

The receiving part is a substantially cylindrical one piece part and has a top end 51 and a bottom end 52. A passageway extending from the top end 51 to the bottom end 52 is formed by a coaxial bore 53 followed by a seat portion 54 for receiving the head 4 of the screw member 2. The seat portion 54 has an opening 55 at the bottom end 52 through which the shaft 3 of the screw member 2 extends. The seat portion 54 is shown to be spherically-shaped, but it can be tapered or it can have any other shape that allows to receive the head 4 so that it can pivot with respect to the receiving part 5. At the top end 51 a substantially U-shaped recess 56 is provided by means of which two free legs 57, 58 are formed that are the sidewalls of a channel for receiving the rod 20. An internal thread 59 is provided at the legs 57, 58 for cooperating with the inner screw 7.

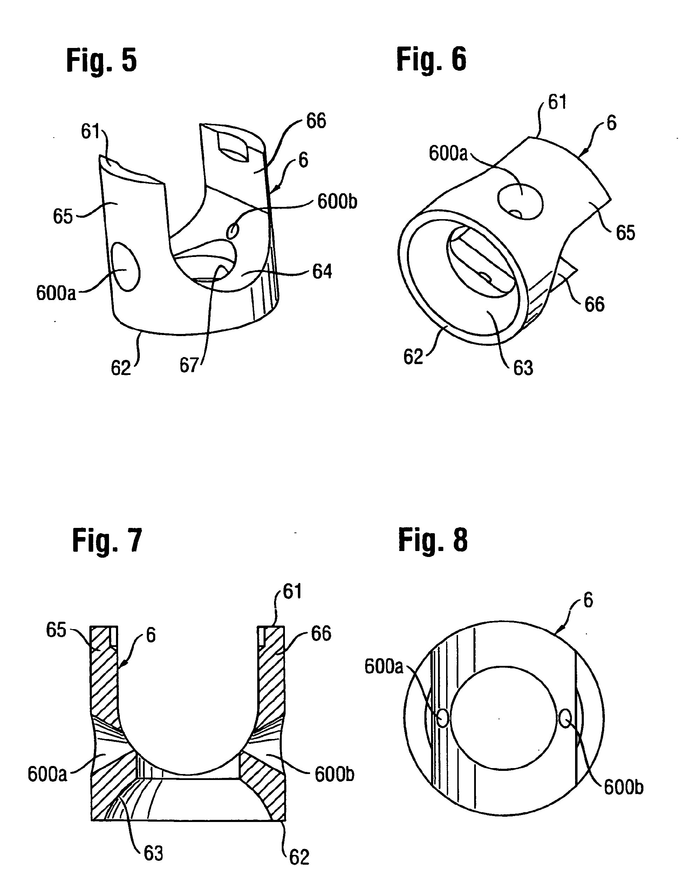

As can be seen from Figs. 5 to 8 the pressure element 6 is formed in one piece. It is of substantially cylindrical construction and has an outer diameter, which allows it to move in the axial direction within the bore 53 of the receiving part 5. The pressure element 6 has a top end 61 and a bottom end 62. When the pressure element 6 is inserted into the receiving part 5, the bottom end 62 faces the head 4 of the screw element 2. At the bottom end 62 a spherical recess 63 is provided, which is adapted to the size and shape of the head 4. The spherical recess 63 is configured to come into frictional engagement with the spherical surface of the head 4. At the top end 61, a U-shaped recess 64 is provided by means of which two free legs 65, 66 are formed that form a channel to receive the rod 20 therein. Furthermore, the pressure element 6 includes a coaxial bore 67 for accessing the screw head 4 with a tool (not shown). The pressure element 6 is a solid member without any spring portions, which could render it flexible. It is arranged in the receiving part 5 such that the U-shaped recess 56 of the receiving part 5 and the U-shaped recess 64 of the pressure element 6 are aligned.

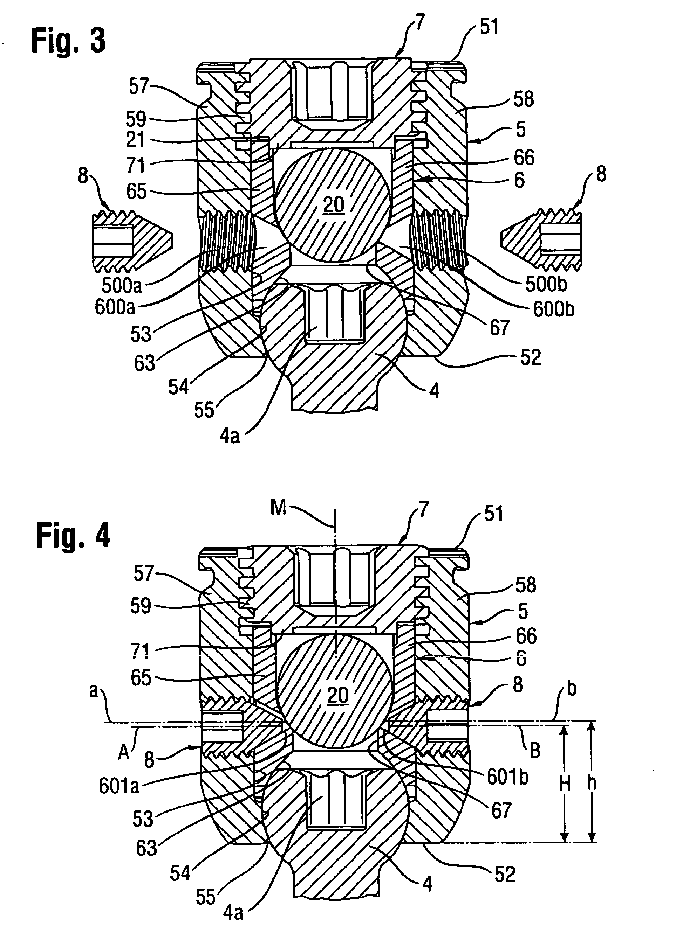

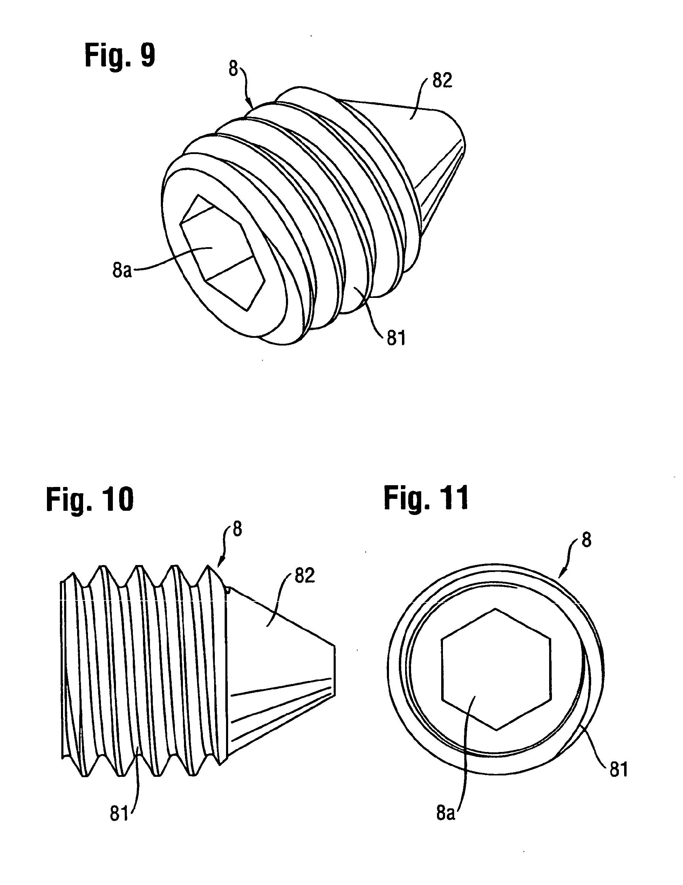

In Fig. 3, the screw head 4 is located in the seat 54 and the pressure element 6 is arranged on top of the screw head 4. The height of the free legs 65, 66 of the pressure element 6 is configured such that the free legs 65, 66 extend above the rod 20 when the rod 20 is inserted and rests on the bottom of the channel. The two set screws 8 are in a non-assembled state. In Fig. 4 the two set screws 8 are in an assembled state. As can be seen from Figs. 9 to 11 the set screw (grub screw) 8 is a headless screw having an outer thread 81, a first end and a second end. On the first end a engagement structure 8a for engagement with a tool (not shown) like a screw driver is provided. In Figs. 9 and 11 it can be seen that the engagement structure 8a is an internal hexagon for engagement with an hexagon socket screw key (not shown). The second end of the set screw 8 is a truncated cone- or taper-shaped portion 82 having a flattened tip.

The locking device 7 in the form of the inner screw 7 has a projection 71 extending into the channel formed by the free legs 65, 66 of the pressure element 6. The size of the projection 71 in an axial direction is such that when the inner screw 7 is tightened, the projection 71 presses onto the rod 20 while there is still a gap 21 between the top end 61 of the pressure element and the lower side of the inner screw 7. Therefore, with the single inner screw 7 pressure can be exerted onto the rod 20 only, which in turn can exert pressure onto the pressure element 6. It should be noted that instead of the single part locking device in form of the inner screw 7 a two-part locking device can be used (not shown). The two-part locking device includes a first part to be screwed in-between the legs 57, 58 of the receiving part. The first part acts onto the top end 61 of the pressure element 6. Further, a second part in form of an inner screw is provided in the first part, which presses onto the rod 20. By means of this, the head 4 and the rod 5 can be independently fixed.

The receiving part 5 includes two threaded through bore holes 500a, 500b extending from the outer surface into the coaxial bore 53. The bore holes 500a, 500b are arranged at 180° offset from each other and at 90° with respect to the channel formed by the U-shaped recess 56. The bore holes 500a, 500b are aligned perpendicular with respect to the bore axis M of the coaxial bore 53. The bore axes A and B of the bore holes 500a, 500b are provided at a distance H from the second end 52 of the receiving part 5.

The pressure element 6 correspondingly includes two through bore holes 600a, 600b which are 180° offset from each other and 90° offset from the channel formed by the U-shaped recess 64. The bore holes 600a, 600b have a center axis a, b, respectively, which is perpendicular to the bore axis M. In the embodiment shown, the bore holes 600a, 600b have a conical shape. The downwardly extending flanks 601a, 601b of the bore holes 600a, 600b each include an angle β of approximately 60° with the central bore axis M. When the pressure element 6 is inserted such that it rests on the head 4 of the screw element, the central axis a, b of the bore holes 600a, 600b has a distance h from the second end 52 of the receiving part 5 that is greater than the distance H of the central axis A, B of the bore holes 500a, 500b. In other words, the bore holes 600a, 600b are arranged above the threaded bore holes 500a, 500b. The bore holes 600a, 600b can also be blind holes facing the inner wall of the receiving part 5 with their open sides.

The distance between the bore holes 600a, 600b and the bore holes 500a, 500b is such that when the set screws 8 are screwed into the bore holes 500a, 500b the taper flanks of the set screws 8 press onto the lower flanks 601a, 601b of the bore holes 600a, 600b, respectively, to exert a downward force onto the pressure element 6. The resulting force onto the pressure element 6 generates a preload onto the head 4, which clamps the head 4 by means of friction. By selecting the thread turning, a desired friction force can be achieved. The screwing-in process can be force-actuated and/or path-controlled. By this friction force the head 4 can be maintained in a desired angular position and can be moved out of this position by applying a force greater than the friction force either to the screw member 2 or to the receiving part 5. Simultaneously, the pressure element 6 is secured against rotation and secured against escaping through the top end 51 of the receiving part 5. The bore holes 600a, 600b provide space for the cones of the set screws 8 when the pressure element 6 moves downward to finally lock the head 4.

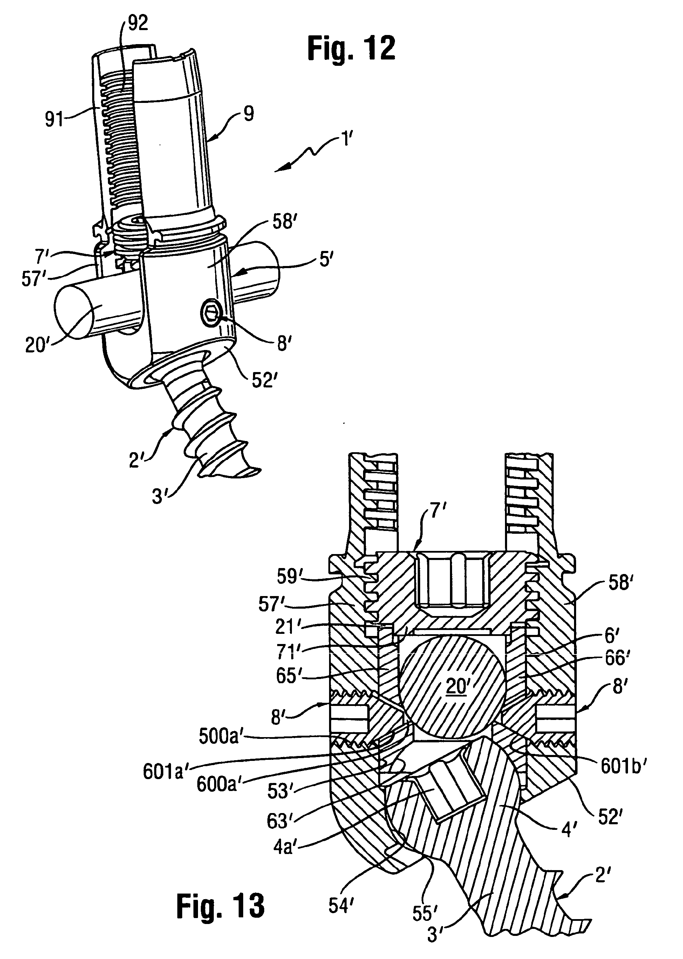

A second embodiment is shown in Figs. 12 and 13. The only difference referring to the first embodiment is the shape of the receiving part 5'. The other parts of the bone anchoring device 1' are the same as that of the first embodiment and the description thereof shall not be repeated.

The receiving part 5' has an asymmetric bottom end 52' for allowing a greater pivot angle of the screw member to one side. Such asymmetric bottom end 52' can be achieved, for example, by cutting away a portion of the receiving part 5' at an inclined angle.

Furthermore, the receiving part 5' is formed with a coaxial tube shaped extension 9, that has a slot 91 for inserting the rod 20' and an internal thread 92. The extension is used for minimally invasive surgery and can be broken away after tightening the inner screw 7'.

It shall be noted that the shape of the bore holes 500a, 500b may vary. In particular, the angle of the cone may vary or the bottom may have rounded or other shape. The bore holes 600a, 600b provided at the pressure element 6 may also have a different shape.

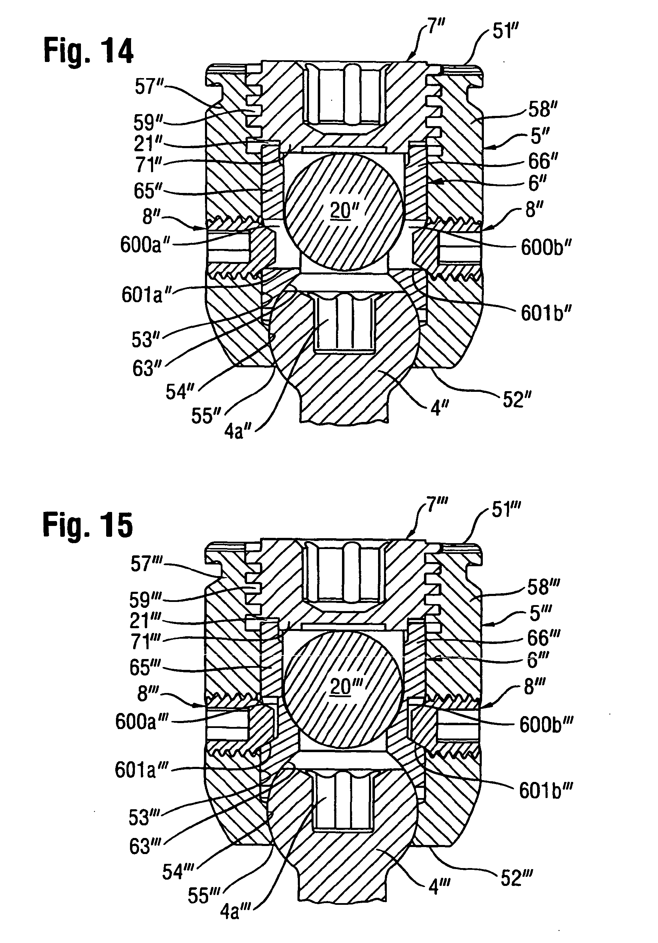

According to a third embodiment shown in Fig. 14, the bore holes 600a", 600b" can have, for example, a substantially rectangular cross-section.

According to a fourth embodiment shown in Fig. 15, the cross-section of the bore holes 600a"', 600b'" of the pressure element 6"' can be for example trapezoidal, with an inclined lower flank 601a"', 601b"' for engagement with the truncated taper flanks of the set screws 8"'.

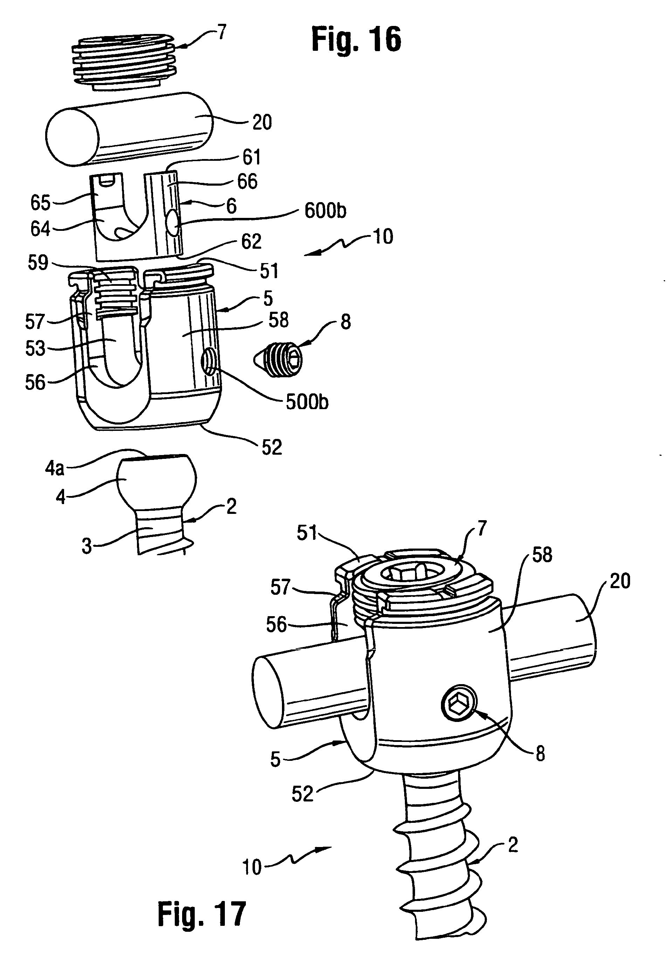

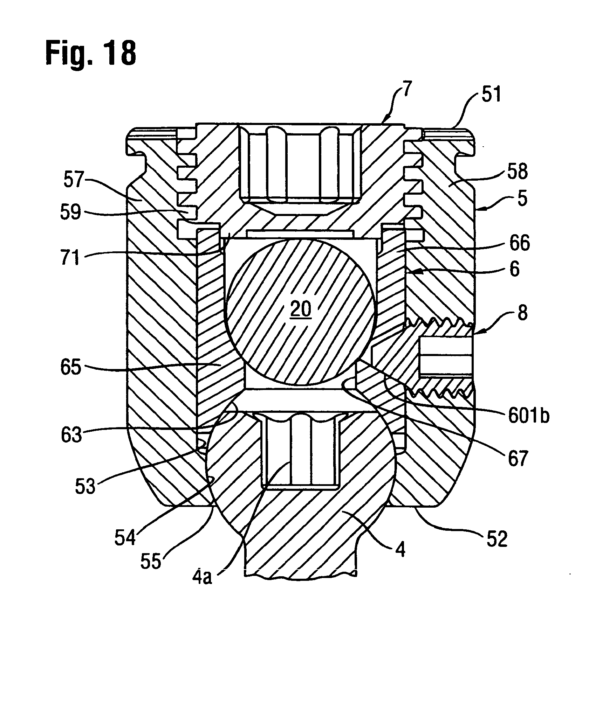

In a fifth embodiment shown in Figs. 16 to 18 a bone anchoring device 10 only comprises a single set screw 8 instead of two set screws 8 as described in the embodiments shown in Figs. 1 to 4 and 14, 15.

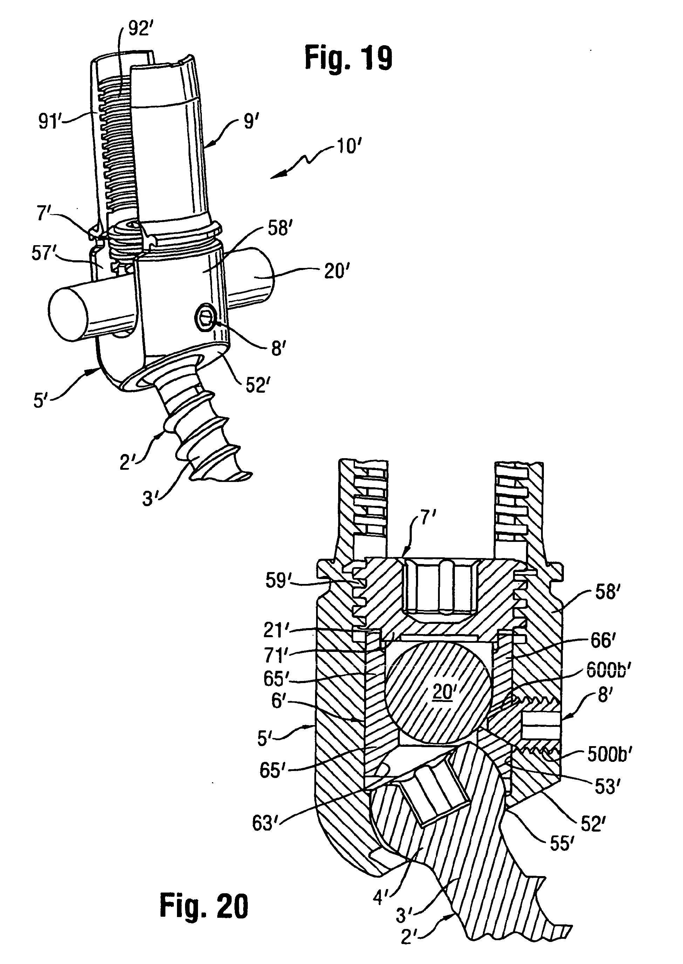

In a sixth embodiment shown in Figs. 19 and 20 a bone anchoring device 10' also only comprises one set screw 8 instead of two set screws 8 as described in the embodiments shown in Figs 12 and 13. All other parts are the same.

All parts of the bone anchoring device 1 are made of a body-compatible material, such as a body-compatible metal, for example, titanium, body-compatible metal alloys such as, for example, Nitinol or from a body-compatible plastic material, such as, for example, polyether ether ketone (PEEK) or combinations thereof.

Usually, several bone anchoring devices 1 are necessary for stabilizing bone parts or vertebrae with the rod. In use, the bone anchoring devices are pre-assembled as shown in Fig. 4. The screw members 2 are screwed into the bone or a vertebra. Then, the receiving parts 5 are pivoted by applying a force greater than the friction force until each receiving part 5 has the correct orientation for the insertion of the rod 20. Due to the friction force, each receiving part 5 is held in this angular position. Thereafter, the rod 20, which connects the bone anchoring devices 1, is inserted and the set screws 8 are tightened to move the pressure element 6 downwards to lock the head 4 in the seat so that the angular position of the screw member 2 with respect to the receiving part 5 is fixed. The rod 20 is fixed by the inner screw 7.

The shape of the through bores and bore holes of the embodiments described is not limited to the described form. Also the angles of the taper are not limited to the values described. Other shapes are possible that achieve also a downwardly directed force when the set screws are screwed in.

For the anchoring element, all kinds of anchoring elements can be used and combined with a receiving part. These anchoring elements are e.g. screws of different lengths, with different diameters, cannulated screws, screws with different thread forms, nails, hooks, etc. The head and the shaft can be separate parts which are connectable to each other.

The shape of the receiving part is not limited to the embodiment shown. For example it is possible to have a recess allowing the rod to be introduced from the side instead of being introduced from the top or a closed recess through which the rod has to be guided. Various kinds of locking devices, including two- or more-part locking devices, outer nuts, outer caps, bayonet locking devices or others are possible.

In a further modification, the receiving part is configured to allow the introduction of the screw element from the bottom end.

## Claims

1. A polyaxial bone anchoring device including
an anchoring element (2, 2', 2", 2"') having a shaft (3, 3', 3", 3"') for anchoring in a bone and a head (4, 4', 4", 4"');
a receiving part (5, 5', 5", 5'") having a top end (51, 51', 51", 51'") and a bottom end (52, 52', 52", 52"'), a channel (56, 56', 56", 56"') for receiving a rod therein and a passage (53, 53', 53", 53"'; 54, 54', 54", 54"'; 55, 55', 55", 55"') extending from the top end (51, 51', 51", 51'") to the bottom end (52, 52', 52", 52"') and including a seat (54, 54', 54", 54"') for receiving the head (4, 4', 4", 4"');
a pressure element (6, 6', 6", 6"') which is arranged in the passage (53, 53', 53", 53"') and configured to exert pressure onto the head (4, 4', 4", 4"'), wherein the head is pivotable with respect to the receiving part (5, 5', 5", 5"') and can be locked at an angle by means of the pressure element (6, 6', 6", 6"');
at least one set screw (8, 8', 8", 8"');
wherein the pressure element (6, 6', 6", 6"') comprising at least one hole (600a, 600a', 600a", 600a"'; 600b, 600b', 600b", 600b"');
wherein the receiving part (5, 5', 5" ,5"') including at least one threaded through hole (500a, 500a', 500a", 500a"'; 500b, 500b', 500b", 500b"') for receiving said at least one set screw (8, 8', 8", 8"');
wherein the through hole (500a, 500a', 500a", 500a"'; 500b, 500b', 500b", 500b"') and the hole (600a, 600a', 600a", 600a"'; 600b, 600b', 600b", 600b"') are arranged such that by screwing in said at least one set screw (8, 8', 8", 8"') into the through hole (500a, 500a', 500a", 500a"'; 500b, 500b', 500b", 500b"') and engagement of said at least one set screw (8, 8', 8", 8"') with the hole (600a, 600a', 600a", 600a"'; 600b, 600b', 600b", 600b"') a force is exerted by the pressure element (6, 6', 6", 6"') onto the head (4, 4', 4", 4'") that maintains the head at an angular position by friction before locking the polyaxial bone anchoring device.

2. The polyaxial bone anchoring device according to claim 1, wherein, when the pressure element (6, 6', 6", 6"') is inserted into the receiving part (5, 5', 5", 5"') and rests upon the head (4, 4', 4", 4"'), the hole (600a, 600a', 600a", 600a"'; 600b, 600b', 600b", 600b"') has a lower edge (601 a, 601b) that is in contact with the set screw (8, 8', 8", 8"') in an axial direction towards the second end (52, 52', 52", 52"').

3. The polyaxial bone anchoring device according to claim 2, wherein the distance in axial direction of an axis M between a center axis of the set screw (8, 8', 8", 8"') and the hole (600a, 600a', 600a", 600a"'; 600b, 600b', 600b", 600b"') is such that the set screw (8, 8', 8", 8'") exerts a pressure onto the pressure element (6, 6', 6", 6'"), when engaging the lower edge (601 a, 601b) of the hole to generate a defined friction force between the head (4, 4', 4", 4"') and the pressure element (6, 6', 6", 6"').

4. The polyaxial bone anchoring device of one of the claims 1 to 3, wherein the bore (600a, 600a'; 600b, 600b') is tapered and wherein the flanks of the taper include the same angle with the axis M as the angle of the flanks of the taper of the set screw (8, 8') with the axis M.

5. The polyaxial bone anchoring device according to claim 4, wherein the angle is between 30 and 60°.

6. The polyaxial bone anchoring device according to one of the claims 1 to 5, wherein the bore (600a", 600b") is cylindrical.

7. The polyaxial bone anchoring device according to one of the claims 1 to 6, wherein the cross-section of the bore (600a"', 600b"') is trapezoidal.

8. The polyaxial bone anchoring device according to one of the claims 1 to 7, wherein at least two through bores (500a, 500a', 500a", 500a"'; 500b, 500b', 500b", 500b'") and corresponding holes (600a, 600a', 600a", 600a"'; 600b, 600b', 600b", 600b"') are provided, which are offset by 180° in a circumferential direction.

9. The polyaxial bone anchoring device according to one of the claims 1 to 8, wherein the pressure element (6, 6', 6", 6'") includes a top end (61, 61', 61", 61'") and a bottom end (62, 62', 62", 62"'), a coaxial bore (67, 67', 67", 67"'), a spherical recess (63, 63', 63" 63"') at the bottom end and a cylindrical or U-shaped recess (64, 64', 64", 64"') at the top end and wherein the cylindrical or U-shaped recess (64, 64', 64", 64"') is aligned with the recess (56, 56', 56", 56"') of the receiving part (5, 5', 5", 5"').

10. The polyaxial bone anchoring device according to one of the claims 1 to 9, wherein the receiving part (5') can have an asymmetric end portion for allowing a greater pivot angle of the anchoring element (2') to one side.

11. The polyaxial bone anchoring device according to one of the claims 1 to 10, wherein the set screw (8, 8', 8", 8"') has a threadless front portion (82) engaging the hole (600a, 600a', 600a", 600a"'; 600b, 600b', 600b", 600b"').

12. The polyaxial bone anchoring device according to claim 11, wherein the threadless front portion (82) is tapered.

## Patentansprüche

1. Eine polyaxiale Knochenverankerungsvornchtung, aufweisend:
ein Verankerungselement (2, 2', 2", 2"') mit einem Schaft (3, 3', 3", 3"') zum Verankern in einem Knochen und einem Kopf (4, 4', 4", 4"');
ein Aufnahmeteil (5, 5', 5", 5"') mit einem oberen Ende (51, 51', 51", 51"') und einem unteren Ende (52, 52', 52", 52"'), einem Kanal (56, 56', 56", 56"') zum Aufnehmen eines Stabs darin und einem Durchgang (53, 53', 53", 53"'; 54, 54', 54", 54"'; 55, 55', 55", 55"'), der sich von dem oberen Ende (51, 51', 51 ", 51"') zu dem unteren Ende (52, 52', 52", 52"') erstreckt und einen Sitz (54, 54', 54", 54"') zum Aufnehmen des Kopfs (4, 4', 4", 4'") aufweist;
ein Druckelement (6, 6', 6", 6"'), welches in dem Durchgang (53, 53', 53", 53"') angeordnet und eingerichtet ist, Druck auf den Kopf (4, 4', 4", 4"') auszuüben, wobei der Kopf schwenkbar in Bezug auf das Aufnahmeteil (5, 5', 5", 5"') ist und anhand des Druckelements (6, 6', 6", 6"') geklemmt werden kann;
wenigstens eine Setzschraube (8, 8', 8", 8"');
wobei das Druckelement (6, 6', 6", 6"') wenigstens ein Loch (600a, 600a', 600a", 600a"'; 600b, 600b', 600b", 600b"') umfasst;
wobei das Aufnahmeteil (5, 5', 5", 5"') wenigstens ein mit Gewinde versehenes Durchtrittsloch (500a, 500a', 500a", 500a"'; 500b, 500b', 500b", 500b'") zum Aufnehmen der wenigstens einen Setzschraube (8, 8', 8", 8"') aufweist;
wobei das Durchtrittsloch (500a, 500a', 500a", 500a"'; 500b, 500b', 500b", 500b"') und das Loch (600a, 600a', 600a", 600a"'; 600b, 600b', 600b", 600b"') derart angeordnet sind, dass durch Einschrauben der wenigstens einen Setzschraube (8, 8', 8", 8"') in das Durchtrittsloch (500a, 500a', 500a", 500a"'; 500b, 500b', 500b", 500b"') und durch den Eingriff der wenigstens einen Setzschraube (8, 8', 8", 8'") in das Loch (600a, 600a', 600a", 600a"'; 600b, 600b', 600b", 600b"') eine Kraft durch das Druckelement (6, 6', 6", 6"') auf den Kopf (4, 4', 4", 4"') ausgeübt wird, die den Kopf durch Reibung in einer Winkelposition hält, bevor die polyaxiale Knochenverankerungsvorrichtung geklemmt ist.

2. Die polyaxiale Knochenverankerungsvorrichtung gemäß Anspruch 1, wobei, wenn das Druckelement (6, 6', 6", 6"') in das Aufnahmeteil (5, 5', 5", 5"') eingesetzt ist und auf dem Kopf (4, 4', 4", 4"') aufliegt, das Loch (600a, 600a', 600a", 600a"'; 600b, 600b', 600b", 600b"') eine untere Flanke (600a, 600b) besitzt, die in Kontakt mit der Setzschraube (8, 8', 8", 8"') in einer axialen Richtung in Richtung auf das zweite Ende (52, 52', 52", 52"') steht.

3. Die polyaxiale Knochenverankerungsvorrichtung gemäß Anspruch 2, wobei der Abstand einer Achse M in axialer Richtung zwischen einer Mittenachse der Setzschraube (8, 8', 8", 8"') und des Lochs (600a, 600a', 600a", 600a"'; 600b, 600b', 600b", 600b"') dergestalt ist, dass die Setzschraube (8, 8', 8", 8"') einen Druck auf das Druckelement (6, 6', 6", 6'") ausübt, wenn es mit der unteren Flanke (601a, 601b) des Lochs in Eingriff tritt, um eine definierte Reibungskraft zwischen dem Kopf (4, 4', 4", 4"') und dem Druckelement (6, 6', 6", 6"') zu erzeugen.

4. Die polyaxiale Knochenverankerungsvorrichtung gemäß einem der Ansprüche 1 bis 3, wobei die Bohrung (600a, 600a'; 600b, 600b') eng zuläuft und wobei die Flanken des Zulaufs den gleichen Winkel mit der Achse M aufweisen wie der Winkel der Flanken des Zulaufs der Setzschraube (8, 8') mit der Achse M.

5. Die polyaxiale Knochenverankerungsvorrichtung gemäß Anspruch 4, wobei der Winkel zwischen 30° und 60° beträgt.

6. Die polyaxiale Knochenverankerungsvorrichtung gemäß einem der Ansprüche 1 bis 5, wobei die Bohrung (600a", 600b") zylindrisch ist.

7. Die polyaxiale Knochenverankerungsvornchtung gemäß einem der Ansprüche 1 bis 6, wobei der Querschnitt der Bohrung (600a"', 600b"') trapezoidal ist.

8. Die Knochenverankerungsvorrichtung gemäß einem der Ansprüche 1 bis 7, wobei wenigstens zwei Durchtrittsbohrungen (500a, 500a', 500a", 500a"'; 500b, 500b', 500b", 500b'") und entsprechende Löcher (600a, 600a', 600a", 600a"'; 600b, 600b', 600b", 600b"') vorgesehen sind, welche in einer Umfangsrichtung um 180° versetzt sind.

9. Die polyaxiale Knochenverankerungsvorrichtung gemäß einem der Ansprüche 1 bis 8, wobei das Druckelement (6, 6', 6", 6"') ein oberes Ende (61, 61', 61", 61"') und ein unteres Ende (62, 62', 62", 62"'), eine koaxiale Bohrung (67, 67', 67", 67"'), eine sphärische Ausnehmung (63, 63', 63", 63"') an dem unteren Ende und eine zylindrische oder U-förmige Ausnehmung (64, 64', 64", 64"') an dem oberen Ende aufweist, und wobei die zylindrische oder U-förmige Ausnehmung (64, 64', 64", 64"') in Bezug auf die Ausnehmung (56, 56', 56", 56"') des Aufnahmeteils (5, 5', 5", 5"') ausgerichtet ist.

10. Die polyaxiale Knochenverankerungsvorrichtung gemäß einem der Ansprüche 1 bis 9, wobei das Aufnahmeteil (5') einen asymmetrischen Endabschnitt besitzen kann, der einen größeren Drehwinkel des Verankerungselements (2') zu einer Seite hin ermöglicht.

11. Die polyaxiale Knochenverankerungsvorrichtung gemäß einem der Ansprüche 1 bis 10, wobei die Setzschraube (8, 8', 8", 8"') einen gewindefreien Vorderabschnitt (82) besitzt, der in das Loch (600a, 600a', 600a", 600a"'; 600b, 600b', 600b", 600b"') eingreift.

12. Die polyaxiale Knochenverankerungsvorrichtung gemäß Anspruch 11, wobei der gewindefreie Vorderabschnitt (82) eng zuläuft.

## Revendications

1. Dispositif d'ancrage osseux polyaxial comportant
un élément d'ancrage (2, 2', 2", 2"') ayant un arbre (3, 3', 3", 3"') pour l'ancrage dans un os et une tête (4, 4', 4", 4"') ;
une partie de réception '(5, 5', 5", 5"') ayant une extrémité supérieure (51, 51', 51", 51"') et une extrémité inférieure (52, 52', 52", 52"'), un canal (56, 56', 56", 56"') destiné à recevoir une tige à l'intérieur et un passage (53, 53', 53", 53"' ; 54, 54', 54", 54"'; 55, 55', 55", 55"') s'étendant à partir de l'extrémité supérieure (51, 51', 51", 51"') à l'extrémité inférieure (52, 52', 52", 52"') et comportant un siège (54, 54', 54", 54"') pour recevoir la tête (4, 4', 4", 4"') ;
un élément de pression (6, 6', 6", 6"') qui est disposé dans le passage (53, 53', 53", 53"') et configuré pour exercer une pression sur la tête (4, 4', 4", 4"'), où la tête peut pivoter par rapport à la partie de réception (5, 5', 5", 5"') et peut être bloquée à un angle à l'aide de l'élément de pression (6, 6', 6", 6"') ;
au moins une vis de pression (8, 8', 8", 8"') ;
dans lequel l'élément de pression (6, 6', 6", 6"') comprenant au moins un trou (600a, 600a', 600a", 600a"' ; 600b, 600b', 600b" , 600b"') ;
dans lequel la partie de réception (5, 5', 5", 5"') comportant au moins un trou débouchant fileté (500a, 500a', 500a" , 500a"' ; 500b, 500b', 500b" , 500b"') destiné à recevoir ladite au moins une vis de pression (8, 8', 8", 8"') ;
dans lequel le trou débouchant (500a, 500a', 500a", 500a"'; 500b, 500b', 500b", 500b"') et le trou (600a, 600a', 600a", 600a"' ; 600b, 600b', 600b", 600b"') sont disposés de sorte qu'en vissant ladite au moins une vis de pression (8, 8', 8", 8"') dans le trou débouchant (500a, 500a', 500a", 500a"' ; 500b , 500b', 500b", 500b"') et en engageant ladite au moins une vis de pression (8, 8', 8"_{,} 8"') avec le trou (600a, 600a', 600a", 600a"' ; 600b, 600b', 600b", 600b"') une force soit exercée par l'élément de pression (6, 6', 6", 6"') sur la tête (4, 4', 4", 4"') qui maintient la tête dans une position angulaire par friction avant le verrouillage du dispositif d'ancrage osseux polyaxial.

2. Dispositif d'ancrage osseux polyaxial selon la revendication 1, dans lequel, lorsque l'élément de pression (6, 6', 6"_{,} 6"') est inséré dans la partie de réception (5, 5', 5", 5"') et repose sur la tête (4, 4', 4"_{,} 4"')_{,} le trou (600a, 600a', 600a" , 600a"'; 600b, 600b', 600b", 600b"') présente un bord inférieur (601a, 601b) qui est en contact avec la vis de pression (8, 8', 8", 8"') dans une direction axiale vers la deuxième extrémité (52, 52', 52", 52"').

3. Dispositif d'ancrage osseux polyaxial selon la revendication 2, dans lequel la distance dans la direction axiale d'un axe M entre un axe central de la vis de pression (8, 8', 8", 8"') et le trou (600a, 600a', 600a", 600a"' ; 600b, 600b', 600b" , 600b"') est définie de sorte que la vis de pression (8, 8', 8", 8"') exerce une pression sur l'élément de pression (6, 6', 6", 6"'), lors de l'engagement du bord inférieur (601a, 601b) du trou pour générer une force de frottement définie entre la tête (4, 4', 4", 4"') et l'élément de pression (6, 6', 6", 6"').

4. Dispositif d'ancrage osseux polyaxial selon l'une des revendications 1 à 3, dans lequel l'alésage (600a, 600a' ; 600b, 600b') est conique et où les flancs de la conicité comportent le même angle avec l'axe M que l'angle des flancs de la conicité de la vis de pression (8, 8') avec l'axe M.

5. Dispositif d'ancrage osseux polyaxial selon la revendication 4, dans lequel l'angle varie entre 30 et 60°.

6. Dispositif d'ancrage osseux polyaxial selon l'une quelconque des revendications 1 à 5, dans lequel l'alésage (600a", 600b") est cylindrique.

7. Dispositif d'ancrage osseux polyaxial selon l'une quelconque des revendications 1 à 6, dans lequel la section transversale de l'alésage (600a"', 600b"') est trapézoïdale.

8. Dispositif d'ancrage osseux polyaxial selon l'une quelconque des revendications 1 à 7, dans lequel au moins deux alésages débouchants (500a, 500a', 500a", 500a"' ; 500b, 500b', 500b", 500b"') et des trous correspondants (600a, 600a', 600a", 600a"' ; 600b, 600b', 600b", 600b"') sont prévus, qui sont décalés de 180° dans une direction circonférentielle.

9. Dispositif d'ancrage osseux polyaxial selon l'une quelconque des revendications 1 à 8, dans lequel l'élément de pression (6, 6', 6",6"') comporte une extrémité supérieure (61, 61', 61", 61"') et une extrémité inférieure (62, 62', 62", 62"'), un alésage coaxial (67, 67', 67", 67"'), un évidement sphérique (63, 63', 63", 63"') à l'extrémité inférieure et un évidement cylindrique ou en forme de U (64, 64', 64", 64"') à la partie supérieure et dans lequel l'évidement cylindrique ou en forme de U (64, 64', 64", 64"') est aligné avec l'évidement (56, 56', 56", 56"') de la partie de réception (5, 5', 5", 5"')_{.}

10. Dispositif d'ancrage osseux polyaxial selon l'une quelconque des revendications 1 à 9, dans lequel la partie de réception (5') peut avoir une partie d'extrémité asymétrique pour autoriser un plus grand angle de pivotement à l'élément d'ancrage (2') à un côté.

11. Dispositif d'ancrage osseux polyaxial selon l'une quelconque des revendications 1 à 10, dans lequel la vis de pression (8, 8', 8", 8"') présente une partie avant non filetée (82) s'engageant dans le trou (600a, 600a', 600a", 600a'" ; 600b, 600b', 600b", 600b"').

12. Dispositif d'ancrage osseux polyaxial selon la revendication 11, dans lequel la partie avant non filetée (82) est conique.
